# FASCICULE DE BREVET EUROPEEN

(11) **EP 1 328 205 B1**
(45) Date de publication et mention de la délivrance du brevet: **30.03.2005**
(21) Numéro de dépôt: 01978533.6
(22) Date de dépôt: 15.10.2001
(51) Int. Cl.: A61B 18/02

(54) **APPAREIL A MAIN DE CRYOTHERAPIE PAR PROJECTION**
HANDSPRÜHGERÄT ZUR KRYOTHERAPIE
HAND-HELD APPARATUS FOR SPRAY CRYOTHERAPY

(30) Priorité: 13.10.2000 FR 0013128
(43) Date de publication de la demande: 23.07.2003
(73) Titulaire: CRYONIC MEDICAL, 39110 Salins Les Bains (FR); VALLOUREC COMPOSANTS AUTOMOBILES VITRY, 51300 Vitry le Francois (FR)
(72) Inventeur: CLUZEAU, Christian, F-39110 Salins les Bains (FR); DESBROSSE, Jacky, F-51300 Huiron (FR)
(74) Mandataire: Bruder, Michel
(86) Numéro de dépôt international: PCT/FR2001/003190
(87) Numéro de publication internationale: WO 2002/030309

(56) Documents cités:
- DE-A- 19 548 652
- FR-A- 2 775 589

## Description

L'invention concerne un appareil de cryothérapie par projection autonome et manipulable avec une seule main particulièrement efficace dans le traitement de la douleur, des inflammations, ou encore pour stimuler la circulation : on sait bien que le froid entraîne un réflexe vasomoteur et une intensification de l'action de drainage, voire procure une détente musculaire par abaissement du tonus musculaire : on sait bien que le froid permet d'obtenir une réponse réflexe.

A cet égard, il est rappelé que le froid est beaucoup plus efficace que la chaleur à la condition toutefois de traiter avec un froid intense qui doit en outre provoquer une chute de température de la zone à traiter pour amener l'épiderme de sa température normale (environ 32°C) à une température comprise entre 2°C et 5°C où l'action du froid est maximale sur les récepteurs cutanés. Accessoirement, on a constaté que l'efficacité de l'action thermique du gaz projeté en phase liquide est encore accrue dans des proportions importantes par l'action complémentaire de la pression qu'exerce le flux sur l'épiderme. La plupart des appareils de cryothérapie utilisent comme source de froid soit l'air réfrigéré, soit plus généralement l'azote liquide dont la mise en oeuvre reste soumise à de nombreuses contraintes limitant le plus souvent la mobilité de l'appareil de traitement ; dans le domaine du sport où la cryothérapie trouve une de ces applications majeures, on utilise couramment de petites bombes de gaz sous basse pression, dont la détente aux abords d'un traumatisme local produit un froid n'excédant pas - 25°C avec une chute de température et une vitesse de refroidissement insuffisantes.

En revanche, il a déjà été proposé dans le brevet européen EP-0.633.008 au nom du demandeur, d'utiliser comme source de froid pour la cryothérapie, de l'anhydride carbonique (CO₂) comprimé dont une caractéristique intrinsèque est de procurer une température de détente de - 78°C à la pression atmosphérique. Selon les enseignements de ce brevet, l'utilisation d'anhydride carbonique sous forme d'un mélange liquide/vapeur permet de maintenir une pression constante dans le récipient pendant sa vidange ; cette pression qui n'est autre que la pression de vapeur saturante dépend directement de la température du récipient. Ainsi, en se détendant jusqu'à la pression atmosphérique, l'anhydride carbonique se trouve sous deux phases : solide et gazeuse. C'est la phase solide, également appelée carboglace, qui permet de soutirer le maximum de calories au niveau de l'épiderme du patient à traiter. En effet, à son contact, la carboglace se sublime, évacuant ainsi une grande quantité de chaleur. Il est donc essentiel de soutirer du récipient la phase liquide de l'anhydride carbonique pour obtenir à la fois un froid intense et une chute de température la plus rapide possible.

Il a déjà été proposé dans le brevet FR-2.775.589 au nom du demandeur, un appareil autonome, léger et portable, susceptible, de produire un froid intense procurant une chute de température très rapide, et ce, dans des conditions de sécurité thermique maximales utilisant à titre principal la température de détente (de l'ordre de - 78°C à la pression atmosphérique) de l'anhydride carbonique (CO₂ ) ou équivalent en phase liquide/solide ; pour cela, l'appareil comporte un réservoir de CO₂ liquéfié sous pression dont la tête est reliée à un système d'éjection et de détente du CO₂ liquide/solide, et comporte des organes de commande du système d'éjection et de contrôle de la température de la zone d'utilisation ; cet appareil est remarquable en ce que la tête du réservoir de CO₂ est agencée sur l'appareil de telle manière que pendant toute la durée d'utilisation, seule la partie liquide du CO₂ à l'intérieur du réservoir vienne au contact de ladite tête pour y être soutirée et propulsée vers la zone d'utilisation.

Cet appareil a été conçu pour un usage par des professionnels avec l'inconvénient qu'il n'est pas utilisable par le particulier dans un contexte familial. La présente invention concerne donc un appareil perfectionné pour être utilisable par tout utilisateur adulte simplement et sans qu'il puisse survenir le moindre danger en cas d'utilisation dans de mauvaises conditions.

A cet égard, il est proposé, conformément à l'invention, un appareil autonome de cryothérapie manipulable avec une seule main, utilisant la température et la pression de détente d'un gaz liquéfié projeté en phase au moins partiellement solide sur un épiderme humain ou animal, tel que du CO₂ ou équivalent, contenu sous pression dans une cartouche amovible, dont la tête de soutirage, qui est reliée par un support à un système commandé d'éjection et de détente du gaz liquéfié, est telle que, pendant toute la durée d'une projection, seule la partie liquide du gaz à l'intérieur de la cartouche vienne au contact de ladite tête de soutirage et cet appareil est remarquable en ce que le support de la cartouche et le système commandé d'éjection et de détente sont disposés sensiblement en ligne et dans l'axe de l'appareil, à l'intérieur d'un boîtier ergonomique d'axe correspondant à celui de l'appareil, dont la partie médiane sert de poignée pour une prise à pleine main de l'utilisateur adulte et comporte l'organe de commande du système commandé d'éjection et de détente du gaz, dont la partie supérieure présente une entrée pour introduire dans son support la cartouche, tête en bas et dans l'axe du boîtier, et la percuter, ou pour la retirer après usage, et dont la partie inférieure comporte une ouverture calibrée formant un passage pour le gaz en projection sensiblement dans l'axe du boîtier et des moyens pour tenir à distance requise la buse de sortie du système commandé d'éjection et de détente par rapport à la zone de l'épiderme à traiter.

Il s'agit donc d'un appareil léger et compact ne pouvant fonctionner dans les conditions de froid intense telles que rappelées en préambule que lorsqu'il est pris à pleine main et qu'il est disposé au droit de la zone à traiter en position quasiment verticale pour obtenir une projection de gaz en phase liquide. A cet égard, toute autre position inclinée aboutirait à projeter le gaz en phase gazeuse, c'est-à-dire à une température sans danger pour l'utilisateur.

Selon plusieurs caractéristiques secondaires qui seront détaillées plus loin, l'appareil conforme à l'invention comprend en outre un certain nombre de dispositifs garantissant au maximum la sécurité d'utilisation ; il s'agit notamment de la forme particulière de l'appareil venant s'inscrire parfaitement dans la paume d'une main fermée, procurant une position obligatoire pour la commande de projection de la ligne cryogénique grâce à une double commande électrique actionnée par deux doigts différents de la main maintenant en position verticale l'appareil ; c'est également aux fins de sécurité d'utilisation que, selon une autre caractéristique importante de l'invention, il est prévu un dispositif de mise à distance correcte de la buse d'éjection du gaz afin qu'il soit absolument impossible de refroidir l'épiderme dans la zone de traitement en-dessous d'une température de 2°C, en tout cas avant que la cartouche de gaz équipant l'appareil ne soit totalement vide. Enfin, et selon une dernière caractéristique, il est prévu de compléter les systèmes de protection précédents par des capteurs de température procurant en temps voulu une alarme pour l'utilisateur et/ou coupant la projection dans l'hypothèse où l'épiderme serait en danger de nécrose, c'est-à-dire refroidi en dessous de 2°C.

D'autres caractéristiques et avantages ressortiront mieux encore de la description d'un appareil de cryothérapie pour grand public conforme à l'invention, donnée ci-dessous à titre d'exemple préféré quoiqu'en aucun cas limitatif en référence aux dessins annexés sur lesquels :
- la figure 1 représente une vue générale de l'appareil conforme à l'invention maintenu verticalement dans la main droite d'un utilisateur, à distance correcte de l'épiderme à traiter,
- la figure 2 représente en élévation et en coupe verticale médiane l'appareil suivant la ligne II/II de la figure 4, montrant la structure interne des organes ainsi que leur position relative dans l'appareil,
- la figure 3 est une vue en élévation et en perspective de l'appareil dont on a enlevé une des demi-coquilles formant l'enveloppe pour montrer les organes internes représentés de manière arrachée mais en position relative de fonctionnement.
- la figure 4 est une vue en élévation de face (proximale) de l'appareil monté,
- la figure 5 est une vue en élévation de l'arrière (distale) de l'appareil monté,
- la figure 6 est une représentation partielle et à plus grande échelle de la chaîne cryogénique montrant le détail de la tête de soutirage d'une cartouche de gaz montée sur son support à l'intérieur de l'appareil, juste avant la percussion de son opercule de fermeture,
- la figure 7 est la même que la figure précédente, sauf que la cartouche est représentée totalement engagée sur son support après percussion de son opercule.

En référence aux figures, l'appareil de cryothérapie 1 qui va être décrit à titre d'exemple non limitatif est particulièrement destiné à un usage dit familial, c'est-à-dire par un utilisateur en principe non professionnel.

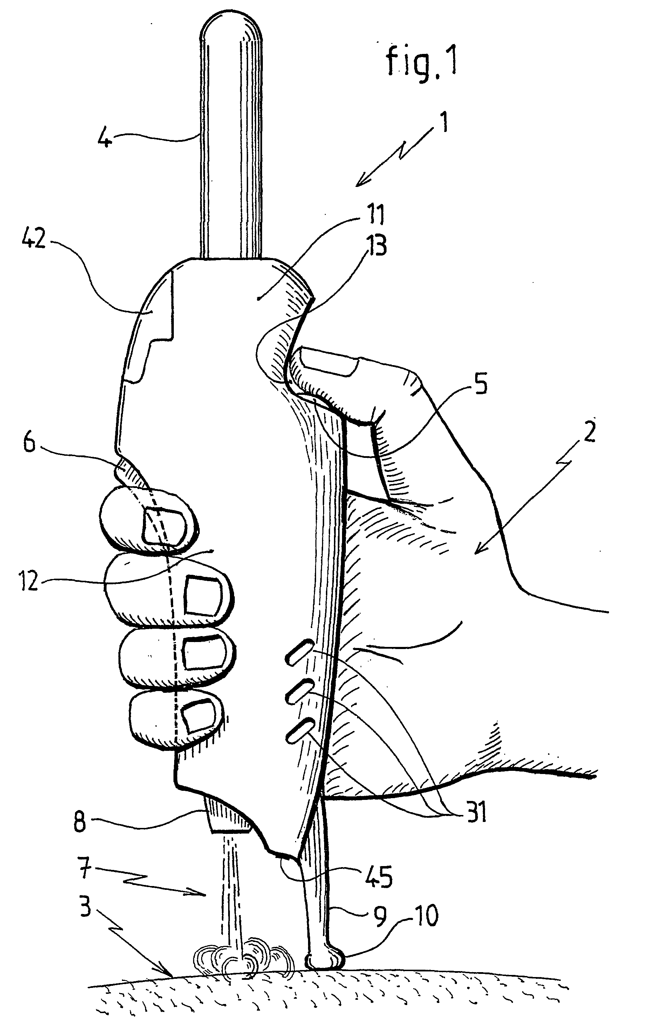

A cet égard, on a représenté sur la figure 1 une vue générale de l'appareil 1 pris par la main droite 2 d'un utilisateur en vue d'une application sur l'épiderme 3 d'une région à traiter, d'un gaz sous pression -préférentiellement de l'anhydride carbonique (CO₂) -, en phase au moins partiellement solide provenant d'une cartouche 4 jetable ou réutilisable, que l'on vient visser, tête en bas, dans un support de cartouche prévu dans la partie supérieure de l'appareil 1 pour maintenir et percuter un opercule d'ouverture mettant en contact le contenu de la cartouche 4 avec la chaîne cryogénique qui sera décrite plus loin. Pour des raisons de sécurité, la projection du gaz sur l'épiderme 3 est commandée par l'action simultané d'un premier interrupteur 5 commandé par le pouce de la main 2 et d'un deuxième interrupteur 6 ayant préférentiellement la forme d'une gâchette-poussoir 6 disposée dans la région de l'index de la main 2 lorsque l'appareil est maintenu à pleine main comme représenté sur la figure 1.

Le CO₂ en phase liquide/solide 7 est éjecté par une ouverture calibrée 8 qui sera précisée plus loin, située en partie inférieure de l'appareil et sensiblement dans son axe vertical. Pour des raisons de sécurité et en particulier pour éviter que la température de l'épiderme 3 en traitement passe en dessous de la température critique de 0°C créant un risque de nécrose de l'épiderme, il est nécessaire de prévoir une distance minimum entre la sortie 8 du gaz et l'épiderme 3 à traiter. Pour ce faire, il est prévu une excroissance en forme de béquille 9 qui s'étend vers le bas sensiblement dans l'axe de l'appareil 1 sur une longueur suffisante pour procurer un écartement convenable entre la sortie 8 et l'épiderme 3 empêchant d'atteindre une valeur critique de température fixée, par exemple à 2°C, lorsque l'on projette l'intégralité du gaz contenu dans la cartouche 4. Il est à noter que l'extrémité de la béquille 9 comporte avantageusement un sabot 10 permettant de répartir la pression de l'appareil sur l'épiderme du patient et éviter de le blesser pendant le traitement ; en outre, la béquille 9 est avantageusement située du côté proximal de l'appareil, c'est-à-dire du côté du bras de l'utilisateur, permettant à celui-ci de mieux visualiser la zone de traitement pendant toute la projection.

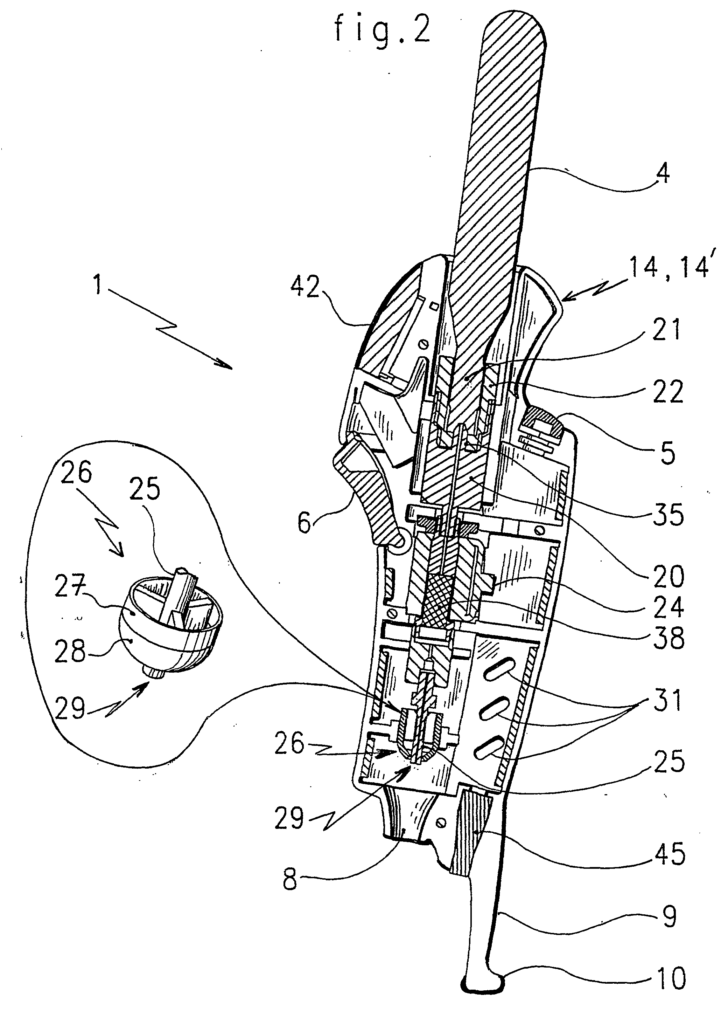

Selon une configuration essentielle de l'appareil 1 conforme à l'invention, la chaîne cryogénique représentée sur la figure 2 est disposée sensiblement en ligne et dans son axe, à l'intérieur d'un boîtier ergonomique 11 dont la partie médiane 12 (figure 1) sert de poignée pour une prise à pleine main d'un utilisateur adulte ; préférentiellement, la partie médiane 12 du boîtier 11 est en fait globalement convexe du côté proximal (c'est-à-dire du côté du bras de l'utilisateur), pour venir épouser la courbure de son pouce ; en outre un évidement 13 dans la partie supérieure de ce côté du boîtier 11, permet un mouvement d'approche et de bascule du pouce de l'utilisateur pour actionner la commande de l'appareil comme il sera dit plus loin sans pour autant rompre le contact de la base du pouce avec le boîtier 11 ; du côté distal (c'est-à-dire du côté le plus éloigné de l'utilisateur) la partie médiane du boîtier est globalement concave pour assurer une prise complète par les quatre autres doigts, procurant ainsi une bonne préhension de l'appareil 1 par une seule main 2 de l'utilisateur adulte et permettre, par l'un ou l'autre de ses quatre doigts, d'actionner simultanément la gâchette 6 pour commander la projection de gaz 7.

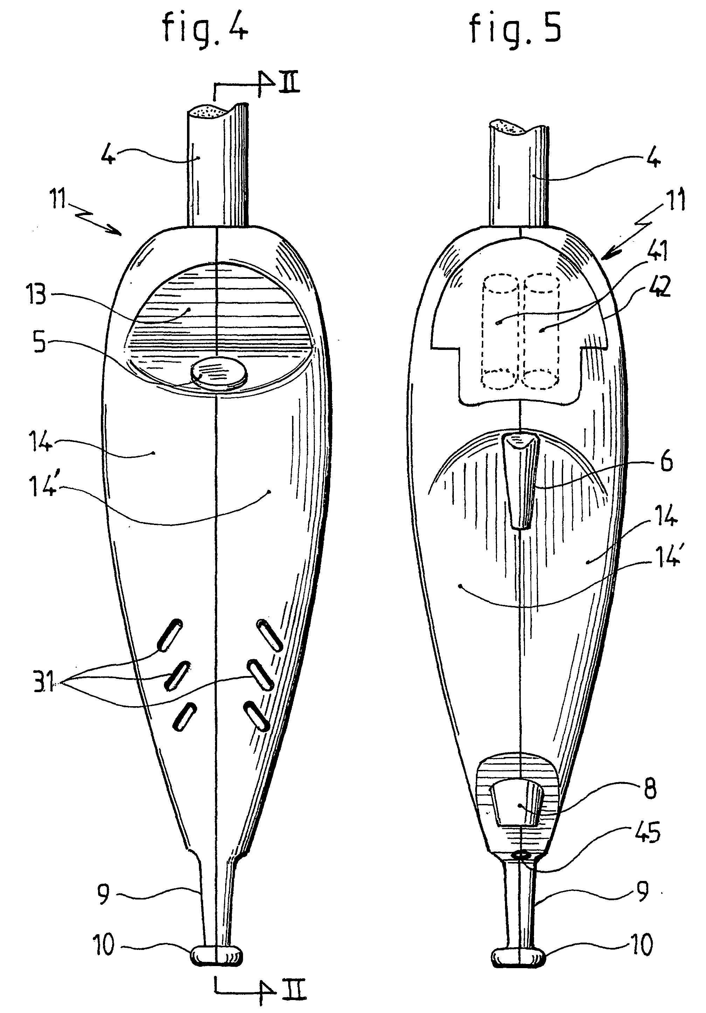

Selon une exécution préférentielle de l'appareil 1, le boîtier 11 est préférentiellement constitué de deux demi-coquilles 14,14' de forme identique, assemblées l'une contre l'autre dans le plan vertical médian du boîtier 11. La forme générale de chaque demi-coquille 14,14' est représentée en plan sur la figure 2, du côté interne au boîtier 11 ; la forme externe de chaque demi-coquille 14,14' est montrée en vue frontale sur la figure 1 et en vues de côté, sur la figure 4 pour la vue proximale et sur la figure 5 pour la vue distale.

Selon une disposition importante de l'appareil conforme à l'invention, la partie médiane 12 du boîtier constituant l'organe de préhension de l'appareil, a une section moyenne telle qu'elle s'adapte parfaitement dans la main d'un adulte mais que réciproquement, elle tienne difficilement dans la main d'un enfant ; ainsi, ce dernier aura-t-il des difficultés à prendre l'appareil avec une seule main, ce qui, en plus des sécurités électriques qui seront précisées plus loin, constitue un élément déterminant pour la sécurité des enfants.

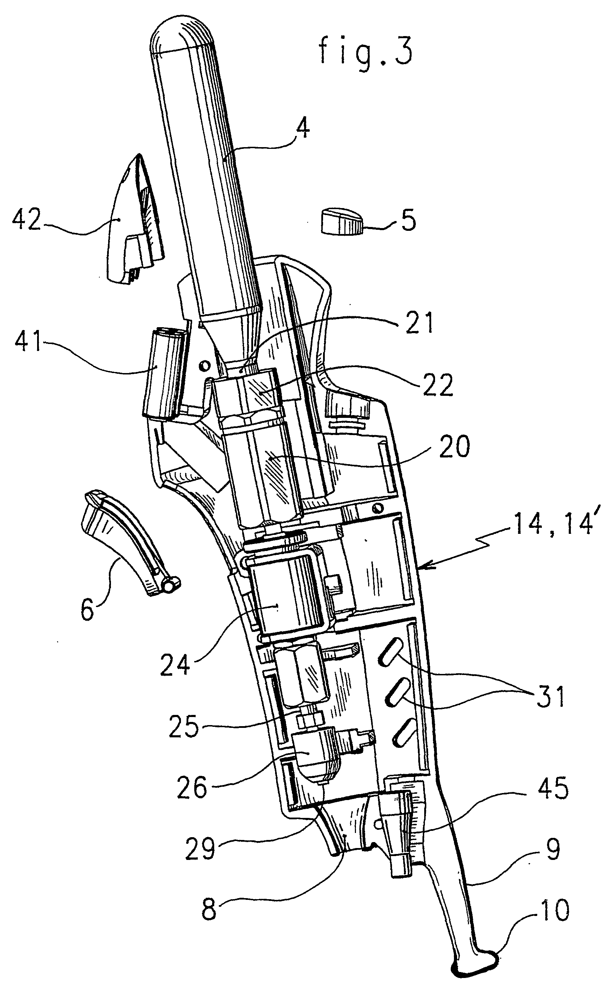

En référence aux figures 2 et 3, il sera maintenant précisé la chaîne cryogénique de l'appareil 1 disposée sensiblement en ligne dans l'axe dudit appareil, et maintenue en bonne position grâce à des logements prévus à l'intérieur des demi-coquilles 14 et 14' dans lesquelles s'encastre juste chacun des éléments constituant la chaîne de sorte qu'après assemblage des deux demi-coquilles 14,14' l'ensemble soit totalement rigide.

La chaîne cryogénique comprend successivement, en partie supérieure de l'appareil 1, un support 20 accueillant du côté supérieur la tête de soutirage 21 de la cartouche 4 par l'intermédiaire d'un écrou 22 formant pièce de liaison et de l'autre côté, un écrou 23 supportant un filtre qui sera précisé plus loin, une électrovanne en ligne 24 destinée à mettre en communication le gaz sous sa forme liquide provenant de la cartouche 4 avec le système d'éjection et de détente du gaz constitué d'une tubulure 25 de faible diamètre intérieur de l'ordre de 0,5 mm. Selon une caractéristique importante de l'appareil 1 conforme à l'invention, il est prévu pour les questions de sécurité déjà abordées en préambule, de contrôler thermiquement le gaz projeté ; pour ce faire, la tubulure 25 du système d'éjection et de détente est associée à un manchon cylindro-conique 26 représenté sur la figure 2 suivant un arrachement à plus grande échelle ; le manchon 26 est monté coaxialement grâce à un calage radial formé d'au moins trois ailettes de manière à ce que la partie cylindrique 27 ayant un diamètre interne plus grand que la tubulure 25, crée un passage d'air tout autour de ladite tubulure 25 ; le sommet de la partie tronconique 28, prolongeant vers le bas la partie cylindrique 27, vient affleurer l'extrémité de la tubulure 25 formant la buse 29 d'éjection du gaz, en ménageant un espace annulaire 30 pour le passage d'air nettement plus faible que dans la partie cylindrique 27 pour créer par effet VENTURI une aspiration de l'air ambiant au travers de lumières 31 aménagées à la base du boîtier 11 afin de limiter de cette manière la température et la pression dans la région de l'épiderme 3 à traiter.

Selon une autre exécution de l'invention, il est également possible de contrôler la température et la pression de sortie du système d'éjection et de détente du gaz en donnant à l'ouverture calibrée 8 en partie inférieure du boîtier 11 une forme générale de tuyère, par exemple en tulipe évasée en direction de la buse 29 et disposée dans le même axe, à bonne distance pour mélanger de l'air ambiant aspiré au travers des lumières 31 par effet VENTURI engendré par la propulsion du gaz.

Il va de soi que l'on peut combiner les deux systèmes de régulation thermique qui viennent d'être décrits pour ajuster plus finement encore la température et la pression du gaz projeté 7.

On décrira maintenant le détail des organes constituant la chaîne cryogénique à l'intérieur de l'appareil, et ce, en référence aux figures 2, 6 et 7.

La cartouche 4 de CO₂, médicalisé le cas échéant, et liquéfié sous une pression d'environ 50 bars, est en fait une cartouche comportant classiquement un récipient métallique de forme cylindrique, fermée à une extrémité, tout à fait analogue mais de taille plus petite, aux cartouches utilisées dans le brevet antérieur FR-2.775.589 déjà cité ; à l'autre extrémité la cartouche est munie d'une tête de soutirage 21 agencée pour coopérer avec un support d'utilisation 20. La tête de soutirage 21 est constituée d'un embout cylindrique fileté extérieurement, d'un opercule 401 qui est destiné à fermer la cartouche avant sa mise en place dans l'appareil et d'un écrou 22 fileté intérieurement et extérieurement, le filetage intérieur de l'écrou 22 étant vissé sur le filetage de l'embout de la tête de soutirage 21 pour maintenir en place l'opercule 401. La cartouche 4 est en principe jetable compte tenu de son utilisation particulière mais pourrait être aussi bien rechargée le cas échéant ; les cartouches contiennent du CO₂ en quantité suffisante pour un traitement d'environ 15 secondes. Le système de soutirage comprend, comme dans d'art antérieur, un système de percussion automatique de la cartouche, assurant la nécessaire étanchéité pendant et après la perforation de l'opercule 401 par exemple en bronze, obturant la cartouche 4. De même, le pas de vis de l'écrou 22 qui vient se visser au-dessus de la tête de soutirage 21 de la cartouche 4 a été volontairement choisi parmi des pas peu courants, de manière à ce qu'il ne soit pas possible d'utiliser des cartouches non conformes.

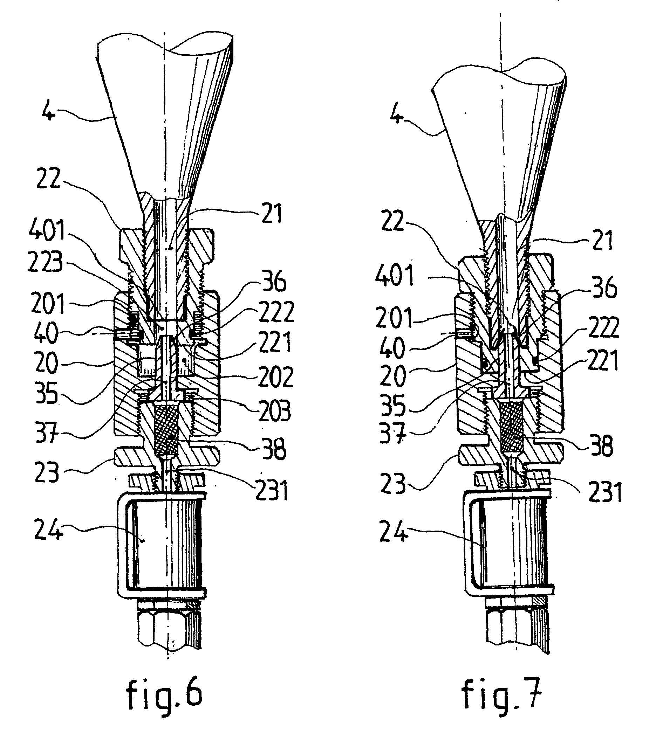

Le système de percussion des cartouches 4 se décompose en trois parties conformément aux figures 6 et 7. Un support cylindrique 20 comportant du côté cartouche une première chambre d'entrée 201 filetée intérieurement dans laquelle vient se visser l'écrou 22 préalablement montée sur la tête de soutirage 21 obturée par l'opercule en bronze 401 ; dans l'axe de cette première chambre 201 et y débouchant, une deuxième chambre 202, de diamètre plus petit, vient collaborer avec l'extrémité 221 de l'écrou 22 de diamètre plus faible que la partie filetée dudit écrou afin d'assurer une étanchéité totale, grâce à un joint torique extérieur 222 monté aux abords de ladite extrémité 221, entre la cartouche 4 et le reste du dispositif, dès que le percuteur 35 perfore l'opercule 401. Le percuteur 35 formant la deuxième partie du système est constitué d'une pointe 36 et est solidaire du support 20 qui peut s'engager dans la partie d'extrémité creuse 223 de l'écrou 22 entourant la tête de soutirage 21 jusqu'à venir au contact de l'opercule 401 lorsque l'engagement dudit écrou 22 dans le support 20 est suffisant pour assurer la solidarisation de l'ensemble ; à cet instant, la pointe 36 est en contact avec l'opercule 401 et le joint torique 222 est au contact des parois de la chambre 202 assurant son étanchéité avec la tête de soutirage 21 ; il suffit alors, comme représenté sur la figure 7, de continuer à visser l'ensemble, cartouche 4/écrou 22 à l'intérieur du support 20 pour perforer l'opercule 401 ; le CO₂ peut alors se détendre vers la partie aval du support 20 en passant d'abord par un canal longitudinal 37 prévu à cet effet dans l'axe du percuteur 35 qui débouche dans une troisième chambre coaxiale 203 du support 20 nécessaire à la mise en place du système de percussion et supportant dans l'écoulement du CO₂ liquide, un filtre 38 destiné à retenir d'éventuels résidus contenus dans la cartouche 4. Le filtre 38 est coiffé par un écrou 23 coopérant avec un filetage interne de la chambre 203 de manière classique. L'écrou 23 est traversé axialement par un canal 231 et il est muni sur sa face aval d'un axe fileté permettant d'implanter à la suite, l'entrée de l'électrovanne 24.

Cette dernière, choisie parmi des électrovannes haute pression, permet l'ouverture et la fermeture d'un canal axial prolongeant le précédent canal 231 par simple commande d'un électro-aimant disposé dans l'axe de l'électrovanne. A la sortie de l'électrovanne 24, on monte le tube de détente 25 se prolongeant, le cas échéant, dans le manchon cylindrico-conique 27 contrôlant thermiquement le flux de CO₂. On notera encore que, jusqu'à la sortie de l'électrovanne 24, le CO₂ est toujours sous sa forme liquide et il n'y a donc pas formation de carboglace susceptible d'empêcher la circulation du fluide ; dans le but de réguler le débit de sortie du CO₂ liquide, on donne au tube de détente 25 un diamètre intérieur très faible, de l'ordre de 0,5 mm ; afin d'éviter la formation d'un bouchon de glace à la sortie du tube de détente, on utilise préférentiellement un tube en PTFE et compte tenu de la pression du CO₂ de l'ordre de 50 bars à la sortie de la cartouche 4, la vitesse d'écoulement du CO₂ à l'intérieur du tube 25 est suffisamment grande pour évacuer à l'extérieur la carboglace en particules micronisées.

Lorsque la cartouche 4 a délivré l'intégralité de son contenu en phase liquide, il est nécessaire de procéder à son remplacement ou selon une autre exécution, à son remplissage. Pour ce faire, il convient de retirer la cartouche 4 de son support 20 et le problème se pose alors d'évacuer préalablement le gaz résiduel à l'intérieur de la cartouche avant son retrait complet, afin d'éviter un recul brusque pouvant être en outre accompagné d'une détonation. Pour purger la cartouche avant son retrait, il est donc prévu un trou de purge 40 reliant l'extérieur de la chambre 202 disposée la plus en amont du support 20, (c'est-à-dire la chambre ne comprenant pas l'organe de percussion 35). Ce trou de purge 40 (figure 6), assure la purge du gaz résiduel au moment où l'on retire la cartouche 4 en dévissant l'écrou 22 de son logement dans la chambre 201 du support 20 ; naturellement, la géométrie intérieure de cette chambre 201 a été prévue pour que, dès que la tête désoperculée de la cartouche 4 se dégage de la pointe de percussion 36, le gaz résiduel soit au contact avec le trou de purge 40, c'est-à-dire avec l'extérieur, alors même que la longueur du filetage de l'écrou 22 encore engagé dans la chambre 201 est encore suffisant pour maintenir rigidement l'ensemble et éviter tout recul brutal de la cartouche libérée de son support ; il suffira donc, dès que le sifflement du gaz au travers du trou de purge 40 apparaît, d'attendre qu'il s'arrête pour continuer à dévisser l'écrou 22 et retirer sans danger la cartouche 4 en vue de son remplacement.

Selon une caractéristique particulièrement importante touchant à la sécurité de l'appareil, l'électrovanne 24 alimentée par des batteries 41 avantageusement disposées dans un logement 42 (figures 3 et 5) dans la partie supérieure de la face distale du boîtier 11 (figure 5) et commandées, c'est-à-dire mises sous tension, par l'appui simultané des deux interrupteurs 5 et 6 montés en série dans l'alimentation électrique, c'est-à-dire entre les batteries 41 et le bobinage de l'électrovanne 24 ; comme il a déjà été dit, et pour éviter toute manipulation notamment par des enfants, la position relative des deux interrupteurs 5 et 6 venant en saillie sur le boîtier 11 est telle que lorsque la partie médiane dudit boîtier est dans la main d'un adulte, le premier interrupteur 5, préférentiellement en forme de bouton poussoir, vienne globalement sous le pouce de l'utilisateur et le deuxième interrupteur 6, avantageusement en forme de gâchette, puisse être actionné par au moins un des quatre autres doigts de la main. On a vu que les deux interrupteurs 5 et 6 se situaient à cet effet respectivement de part et d'autre du boîtier 11 diamétralement opposés dans son plan médian.

Selon une dernière caractéristique de l'invention, l'appareil 1 qui vient d'être décrit peut être avantageusement complété par un organe complémentaire de contrôle de la température de la zone d'utilisation afin d'éviter que l'abaissement de température favorable pour le traitement n'occasionne des nécroses tissulaires ; à cet effet et selon une exécution particulière de l'invention, il est prévu d'adjoindre un détecteur de seuil de température 45 couplé à une électronique de commande et capable de fonctionner à distance sans contact avec la zone d'utilisation ou de soin, conformément aux figures 2 et 3. Placé dans la partie inférieure du boîtier 11 pour que l'optique du détecteur 45 soit centré dans la direction de la zone à traiter, on a choisi de manière préférée, comme détecteur de seuil de température, un pyromètre à infrarouge fonctionnant de préférence avec une alimentation continue de 12 volts comme l'électrovanne 24 ; en pratique, la distance focale de l'optique du pyromètre 45 est choisie de telle sorte que le champ de vision corresponde aussi complètement que possible à la cible à mesurer sur l'épiderme afin d'obtenir une lecture de température juste et précise. A cet effet, la position du pyromètre 45 est justement calculée pour que sa distance à la zone de traitement ne soit ni trop courte car alors le gradient thermique de la peau devient tellement important que la température de la zone traitée et la température de la zone scrutée par le pyromètre sont très éloignées, avec la conséquence de rendre aléatoire la consigne d'alarme, ni trop éloignée ce qui aboutirait à peu près sûrement à une superposition entre le jet de carboglace et le rayonnement infra-rouge, avec la conséquence que le pyromètre relèverait plus sûrement la température du jet que celle de la zone à surveiller.

Selon une caractéristique accessoire, le détecteur de seuil du pyromètre 45 est réglé à une température de l'ordre de 5°C afin de conserver une marge de sécurité ; selon une première exécution, lorsque le seuil de température du pyromètre 45 est atteint, l'électronique de commande qui lui est associée, prévient par un signal sonore ou visuel l'utilisateur et/ou peut couper l'alimentation de l'électrovanne 24, arrêtant brusquement la projection du CO₂ ; selon une autre exécution, le pyromètre 45 peut envoyer un signal sonore ou visuel lorsqu'un premier seuil de danger est atteint et couper l'électrovanne 24 lorsqu'un second seuil est atteint, par exemple aux alentours de 2°C.

L'appareil 1 conforme à l'invention, selon l'une ou l'autre des exécutions qui viennent d'être décrites, pourra être simplement utilisé par n'importe quel utilisateur adulte et non professionnel. Pour cela, il lui suffira d'appréhender par sa main droite par exemple, la partie médiane 12 du boîtier 11 de l'appareil, d'introduire dans sa partie supérieure une cartouche 4 neuve et la viser dans son support 20 jusqu'à obtenir la percussion de son opercule 401 ; l'appareil est alors prêt à fonctionner et il suffira à l'utilisateur adulte de l'approcher de l'épiderme 3 sur lequel on souhaite opérer une cryothérapie en le maintenant verticalement jusqu'à ce que l'extrémité 10 de la béquille 9 soit au contact de la zone à traiter ; à ce moment là et en maintenant verticalement l'appareil, il suffira à l'utilisateur de presser simultanément sur le bouton 5 avec le pouce et sur la gâchette 6 avec son index pour déclencher l'électrovanne 24 et donc la projection 7 d'un flux de CO₂ en phase liquide/solide. En principe, la cartouche de l'appareil est prévue pour une seule opération, c'est-à-dire pour amener la zone à traiter d'une température d'environ 32°C à une température comprise entre 2 et 5°C, et ce, en 15 secondes environ. On a déjà vu précédemment les sécurités thermiques de l'appareil mais on observera néanmoins que lorsque l'appareil est mal positionné volontairement ou pas, c'est-à-dire qu'il n'est plus dans l'axe vertical permettant à la béquille 9 de jouer pleinement son rôle d'écarteur, le diamètre de la cartouche 4 est tel que très rapidement le flux propulsé par la buse 29 du système d'éjection et de détente est en fait sous forme gazeuse donc sans danger du point de vue thermique pour l'épiderme.

Selon enfin une exécution plus simple de l'appareil conforme à l'invention, il est possible de remplacer l'électrovanne 24 et l'ensemble de ses commandes électriques par un dispositif purement mécanique du type vanne mécanique actionnée manuellement. Cette exécution, non représentée sur les figures, consiste à remplacer dans la ligne du froid l'électrovanne par une vanne à piston transversal actionné par un levier disposé dans la zone de préhension du corps de l'appareil ; ainsi, suffit-il de resserrer la gâchette disposée à portée de la main qui tient l'appareil pour actionner la vanne à piston revenant en fait à mettre en contact en amont la tête de soutirage de la cartouche 4 et en aval le système de détente et d'éjection du gaz liquéfié.

Dans cette version simplifiée de l'appareil il va de soi que la tête de soutirage de la cartouche pourra être avantageusement obtenue par un rétreint de l'extrémité de l'enveloppe de la cartouche dotée d'un filetage adéquat venant directement se visser dans le support de cartouche 20 comportant, comme dans la variante précédente, un organe de percussion 35 ; la cartouche ainsi simplifiée est évidemment munie d'un opercule venant coopérer avec ledit organe de percussion, comme dans la variante précédente.

Dans cette exécution, il n'est pas nécessairement prévu de détecteur de température de la zone à traiter dans la mesure où la quantité de gaz contenu dans la cartouche dans sa version simplifiée est telle qu'il ne soit pas possible de descendre en deçà de la température critique même lorsqu'on éjecte totalement le contenu de cette cartouche.

Selon une autre disposition d'ailleurs applicable à toutes les variantes de l'appareil conforme à l'invention, la béquille 9 permettant de disposer à bonne distance la buse 29 du système d'éjection peut être remplacée par un socle évidé de forme cylindrique, tronconique ou encore tronc-pyramidale issu de la partie inférieure du boîtier 11 et s'étendant vers le bas sensiblement dans l'axe dudit boîtier sur une longueur déterminée pour assurer une distance de sécurité suffisante comme expliqué précédemment à propos de la béquille 9. Ce dispositif en socle creux présente en outre l'avantage d'une meilleure stabilité de l'appareil au repos et il évite naturellement toute erreur de positionnement de l'appareil au-dessus de la zone de l'épiderme à traiter.

Le fonctionnement de l'appareil dans sa version mécanique simplifiée est extrêmement simple, ce qui le rend particulièrement adapté pour un usage grand public.

## Revendications

1. Appareil autonome de cryothérapie manipulable avec une seule main, utilisant la température et la pression de détente d'un gaz liquéfié projeté en phase au moins partiellement solide sur un épiderme (3) humain ou animal, tel que du CO₂ ou équivalent, contenu sous pression dans une cartouche (4) amovible, dont la tête de soutirage (21), qui est reliée par un support (20) à un système commandé d'éjection et de détente du gaz liquéfié, est telle que, pendant toute la durée d'une projection, seule la partie liquide du gaz à l'intérieur de la cartouche vienne au contact de ladite tête de soutirage **caractérisé en ce que** le support (20) de la cartouche et le système commandé d'éjection et de détente sont disposés sensiblement en ligne et dans l'axe de l'appareil, à l'intérieur d'un boîtier (11) ergonomique d'axe correspondant à celui de l'appareil, dont la partie médiane sert de poignée pour une prise à pleine main (2) de l'utilisateur adulte et comporte l'organe de commande du système commandé d'éjection et de détente du gaz, dont la partie supérieure présente une entrée pour introduire dans son support la cartouche (4), tête en bas et dans l'axe du boîtier (11), et la percuter, ou pour la retirer après usage, et dont la partie inférieure comporte une ouverture calibrée (8) formant un passage pour le gaz en projection sensiblement dans l'axe du boîtier et des moyens (9) pour tenir à distance requise la buse de sortie (29) du système commandé d'éjection et de détente par rapport à la zone de l'épiderme à traiter.

2. Appareil selon la revendication précédente **caractérisé en ce que** le système commandé d'éjection et de détente comprend une électrovanne (24) en ligne avec une tubulure (25) d'éjection du gaz liquéfié.

3. Appareil selon la revendication 2 **caractérisé en ce que** l'organe de commande de l'ouverture de l'électrovanne (24) est constitué par deux interrupteurs (5,6) actionnables par la main (2) de l'utilisateur et montés en série dans l'alimentation électrique (41) de l'électrovanne (24), l'actionnement de l'électrovanne nécessitant l'appui simultané des deux interrupteurs (5,6) par la main (2) de l'utilisateur.

4. Appareil selon la revendication 3 **caractérisé en ce que** la position relative des deux interrupteurs (5,6) venant en saillie sur le boîtier (11) est telle que, lorsque la partie médiane (12) dudit boîtier est dans la main (2) d'un adulte, le premier interrupteur, par exemple en forme de bouton-poussoir (5), vienne globalement sous le pouce de l'utilisateur et le deuxième interrupteur, avantageusement en forme de gâchette (6), puisse être actionné par un ou plusieurs des quatre autres doigts de la main (2).

5. Appareil selon la revendication 4 **caractérisé en ce que** la partie médiane (12) du boîtier (11) est conformé pour une prise en main totale d'un utilisateur adulte, c'est-à-dire globalement convexe du côté proximal pour suivre la courbure du pouce, un évidement (13) dans la partie supérieure du boîtier (11) permettant le mouvement d'approche du pouce sans rompre le contact de sa base avec le boîtier, et globalement concave du côté distal pour une prise complète des autres doigts.

6. Appareil selon la revendication 1 **caractérisé en ce que** le système commandé d'éjection et de détente comprend une vanne manuelle par exemple à piston disposé transversalement à l'axe de l'appareil, couplée en amont à la tête de soutirage de la cartouche (21) et en aval au système de détente et d'éjection du gaz liquéfié.

7. Appareil selon l'une quelconque des revendications 2 à 6 **caractérisé en ce que** la tubulure (25) du système d'éjection et de détente du gaz liquéfié est une tubulure de faible diamètre intérieur, de l'ordre de 0,5 mm.

8. Appareil selon l'une quelconque des revendications précédentes **caractérisé en ce que** les moyens pour tenir la buse (29) du système d'éjection à distance requise de l'épiderme à traiter (3) sont constitués d'une excroissance du type d'une béquille (9)ou d'un socle évidé de forme cylindrique, tronconique, tronc-pyramidale prévu pour coiffer largement la zone à traiter, issue de la partie inférieure du boîtier (11) et s'étendant vers le bas sensiblement parallèlement à l'axe dudit boîtier sur une longueur déterminée pour tenir la distance requise, lorsque l'appareil (1) est mis en place au droit de l'épiderme (3).

9. Appareil selon l'une quelconque des revendications précédentes **caractérisé en ce que** la cartouche (4) comprend un corps tubulaire fermé à une extrémité et présentant à l'autre extrémité une tête de soutirage (21) constituée d'un embout cylindrique fileté extérieurement, d'un opercule (401) qui est destiné à fermer la cartouche avant sa mise en place dans l'appareil.

10. Appareil selon la revendication 9 avec l'une quelconque des revendications 1 à 5, 7, 8 **caractérisé en ce que** la tête de soutirage (21) de la cartouche est munie d'un un écrou (22) fileté intérieurement et extérieurement, le filetage intérieur de l'écrou (22) étant vissé sur le filetage de l'embout de la tête de soutirage (21) pour maintenir en place l'opercule (401)

11. Appareil selon l'une ou l'autre des revendications 9 ou 10 **caractérisé en ce que** la cartouche (4) est solidarisée au support de cartouche (20) dans l'appareil par vissage de manière à amener d'abord l'opercule (401) au contact d'un organe de percussion (35) avantageusement fixe, s'étendant dans l'axe du support (20), puis à percer ledit opercule (401) en assurant l'étanchéité du montage afin de soutirer le gaz en phase liquide par un canal axial (37) traversant de part en part le dispositif de percussion, relié en aval à son extrémité libre au système commandé de détente, un trou de purge (40) entre l'espace intérieur du support (20) et l'extérieur étant prévu radialement dans la partie dudit support (20) ne comprenant pas l'organe de percussion (35) pour purger le gaz résiduel dans la cartouche vide pendant son dévissage et avant sa complète désolidarisation de l'appareil.

12. Appareil selon l'une quelconque des revendications précédentes **caractérisé en ce qu'**il comprend des moyens de contrôle du gaz projeté qui consistent à entourer la tubulure (25) de sortie du système d'éjection et de détente par un manchon cylindrico-conique (26) monté coaxialement grâce à un calage radial dont la partie cylindrique (27) a un diamètre interne plus grand que la tubulure (25) pour créer un passage d'air tout autour de ladite tubulure et dont le sommet de la partie tronconique (28) prolongeant la partie cylindrique (27) vient affleurer l'extrémité de la tubulure (25) formant la buse d'éjection (29) en ménageant un espace annulaire pour le passage d'air nettement plus faible que dans la partie cylindrique (27), pour créer par effet VENTURI une aspiration de l'air ambiant au travers de lumières (31) aménagées à la base du boîtier (11) et limiter ainsi la température et la pression dans la région à traiter.

13. Appareil selon l'une quelconque des revendications précédentes **caractérisé en ce qu'**il comprend des moyens de contrôle du gaz projeté constitués par la disposition de l'ouverture calibrée (8) en partie inférieure du boîtier (11) a la forme générale d'une tuyère en tulipe évasée en direction de la buse (29) de la tubulure (25) et est disposée dans le même axe, à bonne distance de ladite buse et par des lumières (31) aménagées à la base du boîtier (11) de manière à mélanger au gaz liquéfié en cours de détente de l'air ambiant aspiré au travers de lumières (31) par effet VENTURI créé par la propulsion du gaz.

14. Appareil selon l'une quelconque des revendications précédentes **caractérisé en ce qu'**il comprend un moyen de contrôle thermique de l'épiderme (3) qui est un détecteur de température (45) de l'épiderme (3) agissant sans contact avec celui-ci.

15. Appareil selon la revendication 14 **caractérisé en ce que** le détecteur est un pyromètre à infrarouge (45).

16. Appareil selon la revendication 15 **caractérisé en ce que** la distance focale du pyromètre à infrarouge (45) est telle d'une part qu'il n'y ait pas d'interaction avec la pulvérisation du gaz liquéfié, et d'autre part que la mesure précise de température se fasse lorsque l'appareil (1) est positionné à distance convenable pour l'utilisation envisagée.

17. Appareil selon l'une quelconque des revendications 15 ou 16 **caractérisé en ce que** le pyromètre à infrarouge (45) est couplé à une alarme visuelle et/ou sonore lorsque la baisse de température de la zone d'utilisation atteint un seuil prédéterminé, le pyromètre (45) pouvant alors avantageusement, mais pas nécessairement, couper automatiquement l'alimentation électrique de l'électrovanne (24).

## Claims

1. A self-contained cryotherapy apparatus handleable with only one hand, using the temperature and the expansion pressure of a liquefied gas sprayed in an at least partly solid phase on a human or animal epidermis (3), such as CO₂ or an equivalent gas, contained under pressure in a removable cartridge (4), the discharge head (21) of which being connected through a support (20) to a controlled ejection and expansion system for liquefied gas, is such that, during the whole period for one spray, only the liquid portion of the gas inside the cartridge comes into contact with said discharge head, **characterized in that** the support (20) of the cartridge and the controlled ejection and expansion system are positioned substantially in line with and along the axis of the apparatus, inside an ergonomic case (11) with an axis corresponding to that of the apparatus, the middle portion of which is used as a handle for full-hand gripping (2) by the adult user and includes the controlled gas ejection and expansion system, the upper portion of which has an inlet for inserting the cartridge (4) into its support, upside down and along the axis of the case (11), and for striking it, or for removing it after use, and the lower portion of which includes a calibrated aperture (8) forming a passage for the discharged gas substantially along the axis of the case and means (9) for maintaining the output nozzle (29) of the controlled ejection and expansion system at a required distance from the epidermis area to be treated.

2. The apparatus according to the preceding claim, **characterized in that** the controlled ejection and expansion system comprises a solenoid valve (24) in line with a manifold (25) for ejecting liquefied gas.

3. The apparatus according to claim 2, **characterized in that** the unit for controlling the opening of the solenoid valve (24) consists of two switches (5,6) which may be actuated by the hand (2) of the user and mounted in series in the electrical power supply (41) of the solenoid valve (24), the actuation of the solenoid valve requiring simultaneous pressing of both switches (5, 6) by the hand (2) of the user.

4. The apparatus according to claim 3, **characterized in that** the relative position of both protruding switches (5, 6) on the case (11) is such that when the middle portion (12) of said case is in the hand (2) of an adult, the first switch, for example as a push-button (5), globally comes under the thumb of the user and the second switch, advantageously as a trigger (6), may be actuated by one or more of the four other fingers of the hand (2).

5. The apparatus according to claim 4, **characterized in that** the middle portion (12) of the case (11) is conformed for full-hand gripping by an adult user, i.e., globally convex on the proximal side in order to follow the curvature of the thumb, a recess (13) in the upper portion of the case (11) allowing the approach movement of the thumb without breaking the contact of its base with the case, and globally concave on the distal side for full gripping by the other fingers.

6. The apparatus according to claim 1, **characterized in that** the controlled ejection and expansion system comprises a hand valve, for example with a piston positioned transversely to the axis of the apparatus, coupled upstream with the discharge head of the cartridge (21) and downstream with the liquefied gas expansion and ejection system.

7. The apparatus according to any of claims 2 to 6, **characterized in that** the manifold (25) of the liquefied gas expansion and ejection system is a manifold with a small internal diameter of the order of 0.5 mm.

8. The apparatus according to any of the preceding claims, **characterized in that** the means for maintaining the nozzle (29) of the ejection system at a required distance from the epidermis to be treated (3) consists of a prop (9) type protrusion or a recessed base with a cylindrical, frustro-conical, frustro-pyramidal shape provided for amply capping the area to be treated, from the lower portion of the case (11) and extending downwards substantially parallel to the axis of said case over a determined length in order to maintain the required distance, when the apparatus (1) is placed at right angles to the epidermis (3).

9. The apparatus according to any of the preceding claims, **characterized in that** the cartridge (4) comprises a tubular body closed at one end and having on the other end a discharge head (21) consisting of an externally threaded cylindrical end piece of a cap (401) which is for closing the cartridge before placing it in the apparatus.

10. The apparatus according to claim 9, with any of claims 1 to 5, 7, 8, **characterized in that** the discharge head (21) of the cartridge is provided with an internally and externally threaded nut (22), the internal threading of the nut (22) being screwed onto the threading of the end piece of the discharge head (21) in order to maintain the cap (401) in place.

11. The apparatus according to either of claims 9 or 10, **characterized in that** the cartridge (4) is secured to the cartridge support (20) in the apparatus by screwing, so as to first bring the cap (401) into contact with a striking unit (35), advantageously fixed, extending along the axis of the support (20), and then to pierce said cap (401) while ensuring the sealing off of the assembly in order to discharge the gas in the liquid phase through an axial channel (37) extending right through the striking device, connected upstream at its free end to the controlled expansion system, a purge hole (40) between the inner space of the support (20) and the outside being provided radially in the portion of said support (20) not comprising the striking unit (35) in order to purge the residual gas in the empty cartridge during its unscrewing and before its complete detachment from the apparatus.

12. The apparatus according to any of the preceding claims, **characterized in that** it comprises means for controlling the sprayed gas, which consist of surrounding the exit manifold (25) of the ejection and expansion system with a cylindrico-conical sleeve (26) coaxially mounted by radial keying, the cylindrical portion (27) of which has a larger inner diameter than the manifold (25) in order to create an air passage all around said manifold, and for which the top of the tapered portion (28) extending the cylindrical portion (27) becomes flush with the end of the manifold (25) forming the ejection nozzle (29), by providing an annular gap for the air passage, significantly smaller than in the cylindrical portion (27), in order to create by the Venturi effect, suction of the ambient air through lumens (31) arranged at the base of the case (11) and to thereby limit the temperature and pressure in the region to be treated.

13. The apparatus according to any of the preceding claims, **characterized in that** it comprises means for controlling the sprayed gas formed by the arrangement of the calibrated aperture (8) in the lower portion of the case (11) with the general shape of a tulip nozzle, flared in the direction of the nozzle (29) of the manifold (25) and positioned along the same axis at the right distance from said nozzle and by lumens (31) provided at the base of the case (11) in order to mix the expanding liquefied gas with ambient air sucked in through lumens (31) by the Venturi effect created by the propulsion of the gas.

14. The apparatus according to any of the preceding claims, **characterized in that** it comprises a means for thermally monitoring the epidermis (3) which is a sensor for sensing the temperature (45) of the epidermis (3), acting without any contact with the latter.

15. The apparatus according to claim 14, **characterized in that** the detector is an infrared pyrometer (45).

16. The apparatus according to claim 15, **characterized in that** the focal length of the infrared pyrometer (45) is such that there is no interaction with the spraying of the liquefied gas on the one hand, and on the other hand, an accurate measurement of temperature is performed when the apparatus (1) is positioned at a suitable distance for the contemplated use.

17. The apparatus according to any of claims 15 or 16, **characterized in that** the infrared pyrometer (45) is coupled with a visual and/or sound alarm when the lowering of the temperature of the area of use reaches a predetermined threshold, the pyrometer (45) may then advantageously but not necessarily automatically cut off the electric power supply of the solenoid valve (24).

## Patentansprüche

1. Mit einer Hand bedienbares, autonomes Kryotherapiegerät, die Temperatur und den Entspannungsdruck eines verflüssigten Gases nutzend, das in mindestens teilweise fester Phase auf die Haut (3) eines Menschen oder Tiers gesprüht wird, wie zum Beispiel CO₂ oder ein ähnliches Gas, das unter Druck in einer abnehmbaren Kartusche (4) enthalten ist, deren Entnahmekopf (21), der über einen Halter (20) mit einem gesteuerten Ausstoß- und Entspannungssystem des verflüssigten Gases verbunden ist, so ausgeführt ist, dass während der gesamten Dauer eines Sprühvorgangs nur der flüssige Teil des Gases im Innern der Kartusche mit dem besagten Entnahmekopf in Kontakt kommt, **dadurch gekennzeichnet, dass** der Halter (20) der Kartusche und das gesteuerte Ausstoß- und Entspannungssystem deutlich in einer Reihe und in der Achse des Geräts angeordnet sind, in einem ergonomischen Gehäuse (11), dessen Achse der des Geräts entspricht, dessen Mittelteil als Handgriff dient, damit es von der ganzen Hand (2) eines erwachsenen Anwenders umfasst werden kann, und das Steuerorgan des gesteuerten Ausstoß- und Entspannungssystems des Gases aufweist, dessen oberer Teil einen Eingang aufweist, um in seinen Halter die Kartusche (4) einzusetzen, mit dem Kopf nach unten und in der Achse des Gehäuses (11), und sie aufzuschlagen, oder um sie nach Verwendung abzunehmen, und dessen unterer Teil eine kalibrierte Öffnung (8) aufweist, die einen deutlich in der Achse des Gehäuses liegenden Durchgang für das im Sprühen befindliche Gas bildet, und Mittel (9), um die Ausgangsdüse (29) des gesteuerten Ausstoß- und Entspannungssystems im Verhältnis zum zu behandelnden Hautbereich im erforderlichen Abstand zu halten.

2. Gerät nach vorhergehendem Anspruch, **dadurch gekennzeichnet, dass** das gesteuerte Ausstoß- und Entspannungssystem ein Elektroventil (24) aufweist, dass sich mit einem Rohrstutzen (25) zum Ausstoßen des verflüssigten Gases in einer Reihe befindet.

3. Gerät nach Anspruch 2, **dadurch gekennzeichnet, dass** das Organ zur Steuerung der Öffnung des Elektroventils (24) von zwei Schaltern (5, 6) gebildet wird, die mit der Hand (2) des Anwenders bedienbar und in der elektrischen Versorgung (41) des Elektroventils (24) in Reihe geschaltet sind, wobei zur Betätigung des Elektroventils beide Schalter (5, 6) von der Hand (2) des Anwenders gleichzeitig zu betätigen sind.

4. Gerät nach Anspruch 3, **dadurch gekennzeichnet, dass** die relative Position der beiden Schalter (5, 6), die aus dem Gehäuse (11) herausragen, so ist, dass, wenn sich der Mittelteil (12) des besagten Gehäuses in der Hand (2) eines Erwachsenen befindet, der erste Schalter, der zum Beispiel wie ein Druckknopf (5) ausgebildet ist, im allgemeinen unter dem Daumen des Nutzers liegt, und der zweite Schalter, der vorzugsweise wie ein Drücker (6) ausgebildet ist, mit einem oder mehreren der vier anderen Finger der Hand (2) bedient werden kann.

5. Gerät nach Anspruch 4, **dadurch gekennzeichnet, dass** sich der Mittelteil (12) des Gehäuses (11) dazu eignet, von einem erwachsenen Anwender vollständig mit der Hand umschlossen zu werden, d. h. zur proximalen Seite im allgemeinen konvex ist, um der Krümmung des Daumens zu folgen, wobei eine Aussparung (13) im oberen Teil des Gehäuses (11) die Annäherungsbewegung des Daumens erlaubt, ohne den Kontakt seiner Basis mit dem Gehäuse zu unterbrechen, und zur distalen Seite im allgemeinen konkav ist, um vollständig von den anderen Fingern umfasst zu werden.

6. Gerät nach Anspruch 1, **dadurch gekennzeichnet, dass** das gesteuerte Ausstoß- und Entspannungssystem ein manuelles Ventil aufweist, zum Beispiel mit einem Kolben, der quer zur Achse des Geräts angeordnet, das oberhalb an den Entnahmekopf der Kartusche (21) und unterhalb an das Entspannungs- und Ausstoßsystem für das verflüssigte Gas angeschlossen ist.

7. Gerät nach einem der Ansprüche 2 bis 6, **dadurch gekennzeichnet, dass** der Rohrstutzen (25) des Ausstoß- und Entspannungssystems für das verflüssigte Gas ein Rohr mit einem kleinen Innendurchmesser in der Größenordnung von 0,5 mm ist.

8. Gerät nach einem der vorhergehenden Ansprüche, **dadurch gekennzeichnet, dass** die Mittel, die die Düse (29) des Ausstoßsystems im erforderlichen Abstand zu der zu behandelnden Haut (3) halten, von einer Ausstülpung in der Art eines Stützfußes (9) oder eines hohlen, zylindrischen, kegelstumpfartigen, pyramidenstumpfartigen Sockels gebildet werden, der dazu vorgesehen ist, um den Behandlungsbereich weiträumig abzudecken und aus dem unteren Teil des Gehäuses (11) herausragt und sich deutlich parallel zur Achse des besagten Gehäuses über eine bestimmte Länge nach unten erstreckt, um den erforderlichen Abstand zu halten, wenn das Gerät (1) gegenüber der Haut (3) platziert ist.

9. Gerät nach einem der vorhergehenden Ansprüche, **dadurch gekennzeichnet, dass** die Kartusche (4) einen an einem Ende geschlossenen röhrenförmigen Körper aufweist, der am anderen Ende einen Entnahmekopf (21) hat, der von einem zylindrischen Ansatz mit Außengewinde gebildet wird, einem Verschlussorgan (401), das dazu bestimmt ist, die Kartusche zu verschließen, bevor sie in das Gerät eingesetzt wird.

10. Gerät nach Anspruch 9 mit einem der Ansprüche 1 bis 5, 7, 8, **dadurch gekennzeichnet, dass** der Entnahmekopf (21) der Kartusche mit einer Mutter (22) mit Innen- und Außengewinde versehen ist, wobei das Innengewinde der Mutter (22) auf das Gewinde des Ansatzes des Entnahmekopfes (21) geschraubt wird, um das Verschlussorgan (401) an Ort und Stelle zu halten.

11. Gerät nach Anspruch 9 oder 10, **dadurch gekennzeichnet, dass** die Kartusche (4) mit dem Kartuschenhalter (20) im Gerät durch Verschraubung so verbunden ist, dass zunächst das Verschlussorgan (401) mit einer vorzugsweise feststehenden Zündvorrichtung (35) in Berührung kommt, die sich in der Achse des Halters (20) erstreckt, und danach das besagte Verschlussorgan (401) bei Gewährleistung der Dichtigkeit der Montage durchstoßen wird, um das Gas in flüssiger Phase durch einen axialen Kanal (37) abzuziehen, der die Zündvorrichtung vollständig durchquert, die unterhalb ihres freien Endes mit dem gesteuerten Entspannungssystem verbunden ist, wobei zwischen dem Innenraum des Halters (20) und außen eine Ablassöffnung (40) radial in dem Teil des besagten Halters (20) vorgesehen ist, der die Zündvorrichtung (35) zum Ablassen des überschüssigen Gases in der leeren Kartusche während des Abschraubens und bevor sie vollständig vom Gerät gelöst wird, nicht aufweist.

12. Gerät nach einem der vorhergehenden Ansprüche, **dadurch gekennzeichnet, dass** es Mittel zur Überwachung des gesprühten Gases aufweist, die darin bestehen, dass der Ausgangsstutzen (25) des Ausstoß- und Entspannungssystems von einer zylindrisch-konischen Hülse (26) umgeben wird, die dank einer radialen Fixierung koaxial montiert ist, deren zylindrischer Teil (27) einen größeren Innendurchmesser hat als der Rohrstutzen (25), um einen Luftdurchgang um den besagten Stutzen herum zu schaffen, und deren Spitze des kegelstumpfartigen Teils (28), die den zylindrischen Teil (27) verlängert, genau am Ende des Rohrstutzens (25) anliegt, das die Ausstoßdüse (29) bildet, indem sie für den Luftdurchgang einen kreisförmigen Raum ausbildet, der deutlich kleiner ist als im zylindrischen Teil (27), um durch VENTURI-Effekt Umgebungsluft durch die Schlitze (31), die in die Basis des Gehäuses (11) eingearbeitet sind, anzusaugen und so die Temperatur und den Druck im Behandlungsbereich zu mindern.

13. Gerät nach einem der vorhergehenden Ansprüche, **dadurch gekennzeichnet, dass** es Mittel zur Überwachung des gesprühten Gases aufweist, die aus der Anordnung der kalibrierten Öffnung (8) im unteren Teil des Gehäuses (11) besteht, die allgemein wie ein trichterförmiger, sich in Richtung der Düse (29) des Rohrstutzens (25) erweiternder Rohrstutzen ausgebildet und in der gleiche Achse in einiger Entfernung von der besagten Düse angeordnet ist, und aus Schlitzen (31), die in die Basis des Gehäuses (11) eingearbeitet sind, so das dem verflüssigten, in Entspannung begriffenen Gas Umgebungsluft beigemischt wird, die durch die Schlitze (31) durch VENTURI-Effekt, der durch den Gasvortrieb erzeugt wird, angesaugt wird.

14. Gerät nach einem der vorhergehenden Ansprüche, **dadurch gekennzeichnet, dass** es ein Mittel zur thermischen Überwachung der Haut (3) aufweist, das ein Temperaturfühler (45) der Haut (3) ist, der berührungslos mit dieser funktioniert.

15. Gerät nach Anspruch 14, **dadurch gekennzeichnet, dass** der Fühler ein Infrarot-Pyrometer (45) ist.

16. Gerät nach Anspruch 15, **dadurch gekennzeichnet, dass** die Fokaldistanz des Infrarot-Pyrometers (45) einerseits so ist, dass es zu keiner gegenseitigen Beeinflussung mit der Pulverisierung des verflüssigten Gases kommt und andererseits, dass eine genaue Temperaturmessung erfolgt, wenn das Gerät (1) in angemessener Entfernung für die beabsichtigte Anwendung positioniert ist.

17. Gerät nach einem der Ansprüche 15 oder 16, **dadurch gekennzeichnet, dass** das Infrarot-Pyrometer (45) mit einem visuellen und/oder akustischen Alarm verbunden ist, wenn die Temperatur im Anwendungsbereich so weit abfällt, dass ein vorher festgelegter Schwellenwert erreicht wird, wobei das Pyrometer (45) dann vorteilhafter-, aber nicht notwendigerweise automatisch die Stromversorgung des Elektroventils (24) unterbrechen kann.
